# EUROPEAN PATENT SPECIFICATION

(11) **EP 0 901 354 B1**
(45) Date of publication and mention of the grant of the patent: **17.03.2004**
(21) Application number: 97920889.9
(22) Date of filing: 16.05.1997
(51) Int. Cl.: A61F 2/16

(54) **AN OCULAR IMPLANT**
AUGENIMPLANTAT
IMPLANT OCULAIRE

(30) Priority: 17.05.1996 CH 125596
(43) Date of publication of application: 17.03.1999
(73) Proprietor: Payer, Helmut, 7000 Chur (CH)
(72) Inventor: Payer, Helmut, 7000 Chur (CH)
(74) Representative: Riederer, Conrad A., Dr.
(86) International application number: PCT/IB1997/000567
(87) International publication number: WO 1997/043984

(56) References cited:
- EP-A- 0 064 770
- EP-A- 0 492 126
- WO-A-89/06520
- CH-A- 681 687
- GB-A- 2 151 371
- US-A- 4 878 912
- US-A- 4 994 082
- US-A- 5 026 396
- DATABASE WPI Section PQ, Week 9044 Derwent Publications Ltd., London, GB; Class P, AN 90-333114 XP002021132 & SU 1 553 109 A (EYE DISEASE RES, INST.) cited in the application

## Description

The invention relates to an ocular implant for insertion into the lens capsule of the eye according to the preamble of claim 1.

CH 681687 discloses a plastic lens comprising a flexibly deformable lens member surrounded by a likewise elastically deformable force-transmitting part. The force-transmitting part has approximately the same thickness as the lens and comprises an edge region with two notches for facilitating the insertion of the plastic lens. The force-transmitting part is curved towards the rear, so that the lens has a plate-like shape.

The said plastic lens is based on the idea of using the centripetal forces occurring during the accommodation process to thicken the flexible lens member and thus increase the refractive power thereof.

One disadvantage of the disclosed plastic lens is its relatively large volume, which increases the difficulty of implantation through frontal capsulorhexis. Contrary to original hopes, the lens is non-foldable, because of its volume. This is probably the main reason why the lens has not become important in practice and has not even been produced as a prototype. Another disadvantage is that in the event of progressive fibrotic shrinkage of the lens capsule equator, the lens can be partly pressed through the window in the front of the lens capsule and becomes partly spherical to the rear. As a result of the internal material flow, its refractive power increases. This is shown by increasing myopia, which increasingly incapacitates the patient.

US 4 994 082 discloses a substantially planar intra-ocular implant with a lens having a progressive refractive power and comprising a haptic portion. The disclosed implant, in a first exemplified embodiment, has the advantage that the contraction or relaxation of the ciliary muscle is transmitted by the haptic portion to the lens and is displaced at right angles to the optical axis. The focal point can be altered in dependence on the place where light passes through the lens. In a second exemplified embodiment the optical system comprises two lenses held by three pairs of arms constituting the haptic portion. When the ciliary muscle contracts, one pair of arms spread apart, so that the lenses move apart in the direction of the optical axis. By this means, the refractive power of the optical system can be varied. One disadvantage of the said implants is that they are relatively complicated and cannot be adapted to the accommodation capacity of individual patients. In the first embodiment also, there is a particularly serious risk of the front and the rear lens leaves sticking together.

WO-A-89/06520 describes a planar implant having a lens wherein the ciliary muscle alters the lens curvature and geometry. To this end a deformable central lens structure is provided and comprises two diaphragms which are welded together and form a chamber. The lens is held by a ring structure comprising an inflatable ring. Since the lens and the ring are not inflated until after implantation by filling the chamber with viscous liquid, the implant can be inserted into the eye more easily. Since of course the lens is very easily deformable, the filled ring must ensure that the lens has an adequate yield stress. One disadvantage of the implant is that as fibrous degeneration of the lens-capsule equator increases, the front and rear walls of the lens become curved and, owing to the front capsule window and - after rear capsulotomy - also owing to the rear capsule opening acquire anterior curvature, resulting in incapacitating myopia.

SU 1553-109A describes a crystalline lens supported by a haptic portion formed with peripheral recesses. The lens and the haptic portion are curved, with radii of opposite signs. The implant is inserted so that when the ciliary muscle contracts the lens moves backwards, thus displacing the focal point to the rear (myopia-increasing effect). A disadvantage of the implant is that the front and the rear leaf of the lens capsule may stick together in the region of the recesses, which may result in formation of fibrotic tissue.

The object of the invention is to provide an ocular implant of the kind according to the preamble which substantially maintains the topography of the lens capsule and of the zonule of Zinn, thus avoiding postoperative injury to the eye through the implant. Another object is to improve long sight and near vision by means of the implant. If fibrotic shrinkage of the lens capsule occurs, the lens should not alter, particularly in refractive power or its position relative to the retina, in a manner which increasingly incapacitates the eye.

To this end, an implant according to the preamble of claim 1, comprises a single lens, the haptic portion thereof having an annular edge bead and an annular intermediate region which connects the edge bead to the lens. The annular intermediate region is bent relative to a plane given by the annular edge bed. The intermediate region is constructed so as to be elastically deformable so that when centripetal pressure is exerted on the edge bead there is substantially no deformation of the lens, but instead the lens is moved axially forwardly relative to the edge bead. The advantage of this is that the lens member can move forward under the centripetal pressure of the elastic equatorial ring of the natural lens capsule and makes near accommodation possible. The new plastic lens therefore makes advantageous use of the function, frequently still intact even in older persons, of the ciliary muscle, the fibrils of the zonule of Zinn and the elastic equatorial ring of the lens capsule which has a centripetal effect. This is a means of substantially improving the vision of patients with an inserted intra-ocular lens, so that frequently they have good vision, both distant and near, without additional glasses.

In contrast to the elastically deformable lens according to CH 681687, the invention does not increase myopia, even in the event of progressive fibrosis of the lens capsule, since the lens is practically non-deformable by the forces prevailing in the eye and consequently cannot be pressed through the window in the lens capsule. The remaining fibrotic ring in the front wall of the capsule constitutes a barrier against forward movement of the optical system. It is therefore practically impossible for the patient to be subsequently incapacitated, as may still be the case with the known implant.

The haptic portion is constructed so as to be so elastic that under centripetal force it pushes the lens forward and can expand again when the centripetal force decreases. In the accommodation-free state it holds the lens capsule slightly extended, but during accommodation it can be compressed by the centripetal forces acting in the eye.

The permanent separation between the front and the rear capsule leaf is also important in preventing secondary cataract. This can be permanently ensured by the cap shape of the lens. Cell formation is also inhibited by the surface contact of the haptic portion and the optical system with the inner surface of the equatorial ring of the lens capsule and the remaining front capsule leaf, both of them exclusively representing the germinal zone for cell growth.

The cup shape acts as a spacer for the rear capsule and prevents the front side of the capsule leaf from sticking to the rear. The resulting permanent separation of the capsule leaves is an essential condition for maintaining the elastic forces which must operate during accommodation and de-accommodation.

The annular edge bead or roll firmly supports and automatically centres the implant. The exterior of the bead preferably has a round cross-section. The positive effect of the edge bead is that it can prevent the formation of secondary cataract, since cell growth is prevented by abrasion and by surface contact (annular bead chafes). After an operation, the secondary cataract may occur in only three or four weeks. Previous experience in implantation shows that the proportion of secondary cataracts is particularly low.

The elastic intermediate ring, which is thinner (less material thickness) than the edge bead and the lens in the central region, has the advantage that in the event of extreme and possibly strong fibrosis of the lens capsule equator, the intermediate ring can warp, because the optical system cannot come out of the round window in the front capsule. Also there can be no incapacitating increase in refractive power through thickening of the lens, since the lens is substantially rigid under the forces prevailing in the eye.

The intra-ocular implant according to the invention, comprising an annular bead and having a cup shape, maintains the topography and the geometry of the lens capsule and of the corona ciliaris of the zonule fibrils and is thus the functioning end link in the chain of dynamic components of the accommodation apparatus, so that the remaining function of all the links (ciliary muscle, zonule fibres, elastic equatorial ring of the lens capsule) can be used for increasing pseudo-accommodation.

Preferably the haptic portion is formed with two approximately opposite recesses. The two recesses make it easier for the periphery of the lens to reduce under centripetal force. Also the implant is particularly easy to fold, and this facilitates insertion through the elastic round window created by frontal capsulorhexis. Particularly advantageously, the haptic portion has three recesses. This embodiment has the advantage that the implant, which is folded during the implantation process, can unfold automatically and particularly easily after being released in the lens capsule. Another result of the edge bead is that the lens is automatically centred. In principle more than three recesses or only one recess can be provided.

Advantageously, two recesses are disposed with mirror symmetry at an angle of 120 to 150°, preferably 140°, to the recess at the front in the direction of insertion. The result is a narrower edge bead portion, which can form the tail during the implantation process. Advantageously, undercuts are formed at the ends of the edge beads at the front in the direction of insertion. This can further reduce the resistance of the intermediate region to peripheral reduction of the haptic portion. Since the edge bead forming the tail is not undercut, it cannot be trapped in the elastic front wall of the lens capsule when the implant is inserted through the round window. This is important, because delay or failure of the implantation process may injure the eye.

Preferably the implant is foldable. The folding axis advantageously extends through at least one recess in the case of a single fold. If there is a double fold, at least one recess determines the direction of implantation. The double fold can result in a three-layer implant, the transverse diameter being reduced to little more than a third of the original diameter of the implant. The increase in thickness of the implant, folded in three layers, remains small, owing to the thinness of the intermediate ring. Consequently the width of the incision for implantation in the eye can be reduced to just 4 mm. Implantation is thus possible by the keyhole method, so that the radii of curvature of the cornea are not adversely altered by the operation.

Advantageously, the intermediate region has a number of spaced-apart recesses which facilitate elastic deformation thereof. Alternatively the recesses can be used for improved suction removal of the visco-elastic liquid used during implantation. The perforations can e.g. be circular. The result is a spoke structure.
The perforations may also be rectangular or trapezoidal. However, optimum deformability of the intermediate region can be obtained simply by suitable choice of the elastic properties of the plastic used and of the thickness thereof.

Advantageously, the intermediate region is bent forward between about 5° and 15°, preferably about 10°, relative to a plane at right angles to the optical axis, so that during accommodation the direction of movement of the optically active lens forwards is determined. The implant is well supported by the forwardly bent intermediate region in the outer periphery of the lens capsule. Fibrosis can be prevented by the large surface contact between the peripheral edge bead and the inner surface of the lens capsule equator, and the contact between the edge region of the lens and the edge of the round window in the front capsule. Excessive bending, however, is usually undesired, because this means a risk of chafing the rear pigment leaf of the iris at the edge of the front round window (capsulorhexis edge), which may trigger a pigment dispersion syndrome.

In a particularly advantageous embodiment, the lens has a central zone of increased refractive power or is designed as a multi-focal lens. The optimum automatic centring of the implant enables the near vision to be further assisted by central increase of the power of the lens to the dioptric power necessary for reading. Preferably, the zone of increased refractive power is an approximately circular lens, e.g. from about 0.8 to 2 mm, preferably about 1 mm in diameter. Since the zone of increased refractive power is reduced in size to a minimum, there is no or only inappreciable interference with long sight, which is made possible by the strength of the surrounding lens. During near accommodation the pupil contracts, with the necessary adjustment of convergence of the two eyes on the near object. When the pupil is narrow the path of rays is through the centrally increased-power lens, whereas in the case of desaccommodation to distance the pupil widens and long sight is possible via the surrounding lens having the lower dioptric power. In the physiology of accommodation this means that support of near vision occurs in addition to the dynamic process of accommodation via the more powerful central portion of the lens.

In principle the optical system can be a diffractive lens or a multi-focal lens. It can e.g. comprise concentric rings having different refractive indexes.

Exemplified embodiments of the invention and of folding forceps for folding and holding the lens will now be described with reference to the drawings, in which:
Fig. 1a is a plan view of a first exemplified embodiment of an implant according to the invention having three recesses at the peripheral edge;
Fig. 1b shows the first exemplified embodiment in section;
Fig. 2a shows a second embodiment comprising an additional central lens of higher refractive power and wherein the recesses are partly undercut, likewise in plan view;
Fig. 2b shows the second exemplified embodiment in section;
Fig. 3 shows a third exemplified embodiment of an implant with pairs of opposite recesses and notches in the peripheral edge;
Fig. 4 is a partial cross-section through an eye with a lens fixed in it;
Fig. 5a is a perspective view of a folding forceps for folding the implant;
Fig. 5b is a plan view of the folding forceps in Fig. 5a;
Fig. 6a is a side view of an implant held with the folding forceps;
Fig. 6b shows the same in cross-section;

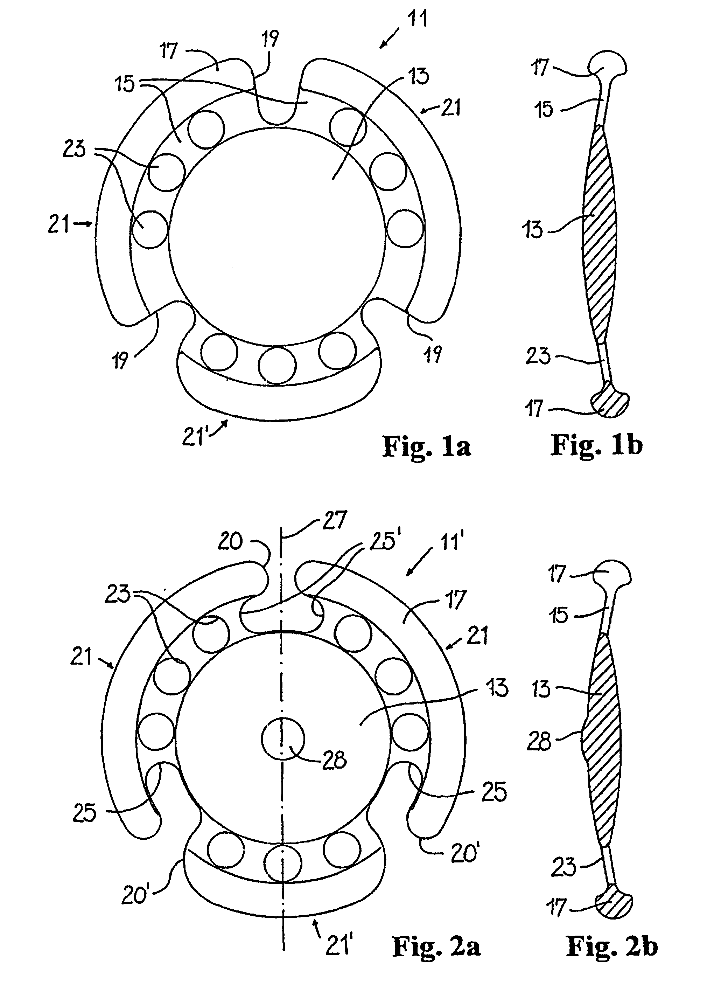

Implants 11, 11' preferably in one piece of suitable plastic, shown in Figs. 1 and 2, comprise a central lens 13 which behaves substantially rigidly after insertion of the implant, i.e. the lense does not change its focal power due to applied centripetal pressure during accomodation, and a haptic portion which surrounds the lens 13 and comprises a peripheral edge bead 17 and an intermediate region 15 which connects the edge bead 17 to the lens 13. The intermediate region 15 is bent forwards, e.g. at an angle of 8 to 12°, preferably about 10°.

The haptic portion is interrupted by three recesses 19 and 20, 20' respectively, resulting in three edge bead portions 21, 21' respectively. The recesses 19 and 20, 20' respectively extend almost to the lens 13 or can be made less deep. By means of the recesses 19, 20 and 20' the periphery or diameter of the haptic portion can be reduced by the centripetal pressure of the ciliary muscle of the eye, with the result that the lens 13 is pushed forward but without being pressed out of the round window in the lens capsule.

The intermediate region 15 has a number of spaced-apart circular recesses 23 which increase the flexibility of the intermediate region 15. In principle the intermediate region 15 should have an elasticity such that under centripetal pressure it can push the lens forward and can expand again when the centripetal pressure decreases. In the optimum case the intermediate region is sufficiently elastic for the intermediate region 15 to bulge forward or backward if there is severe fibrosis of the lens capsule.

The circular recesses 23 result in a spoke structure which ensures the previously-mentioned favourable properties of the intermediate region 15. Other shapes of recesses, e.g. rectangular or trapezoidal, are also possible. The recesses 23 enable the visco-elastic liquid used during the operation to be quickly and completely removed by suction.

Preferably, undercuts 25, 25' are formed adjoining the ends of the edge bead portions 21 at the front in the direction of insertion, thus reducing the resistance to peripheral reduction of the haptic portion under centripetal pressure.

Advantageously, the rear annular portion 21' in the direction of insertion is without such undercuts, to prevent the implant from catching when inserted into the lens capsule.

In the particularly preferred embodiments in Figs. 1 and 2, two recesses 19 and 20, 20' respectively are disposed with mirror symmetry at an angle of approximately 140° to the recess 19, 20 respectively at the front in the direction of insertion. The result is a somewhat narrower edge bead portion 21' which facilitates insertion and unfolding of the implant inserted into the lens capsule. The implant, held by a special folding forceps, can be brought by gentle pressure through the 4 mm section of the cornea into the anterior chamber of the eye and slides like a fish, still folded over about two-thirds of its size, through the elastic round window, created by capsulorhexis, in the front wall of the lens capsule unfolded with visco-elastic substance. When the folding forceps opens, the implant automatically unfolds and centres itself.

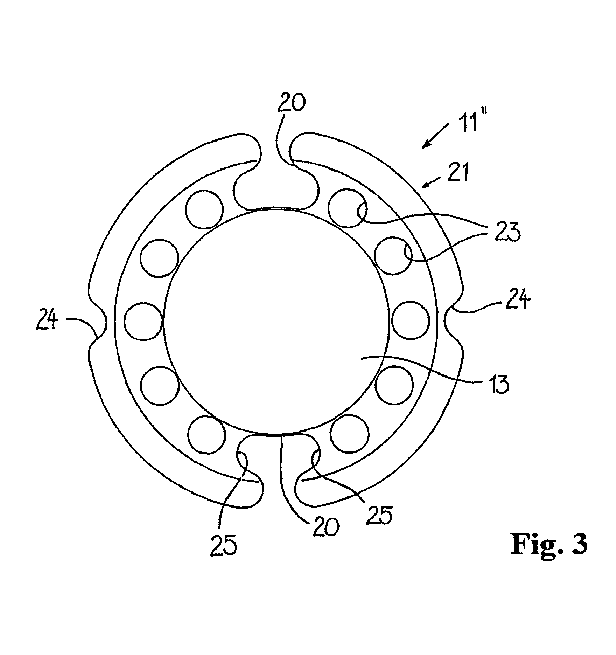

Fig. 3 shows an exemplified embodiment which differs from the previous examples in that instead of three recesses there are only two, having the reference 20. The recesses 20 are diametrically opposite one another. In addition, two likewise diametrically opposite smaller recesses or notches 24 are formed in the edge bead 17. These facilitate implantation when there is only one fold. The recesses 20 and notches 24 are offset from one another by 90° respectively.

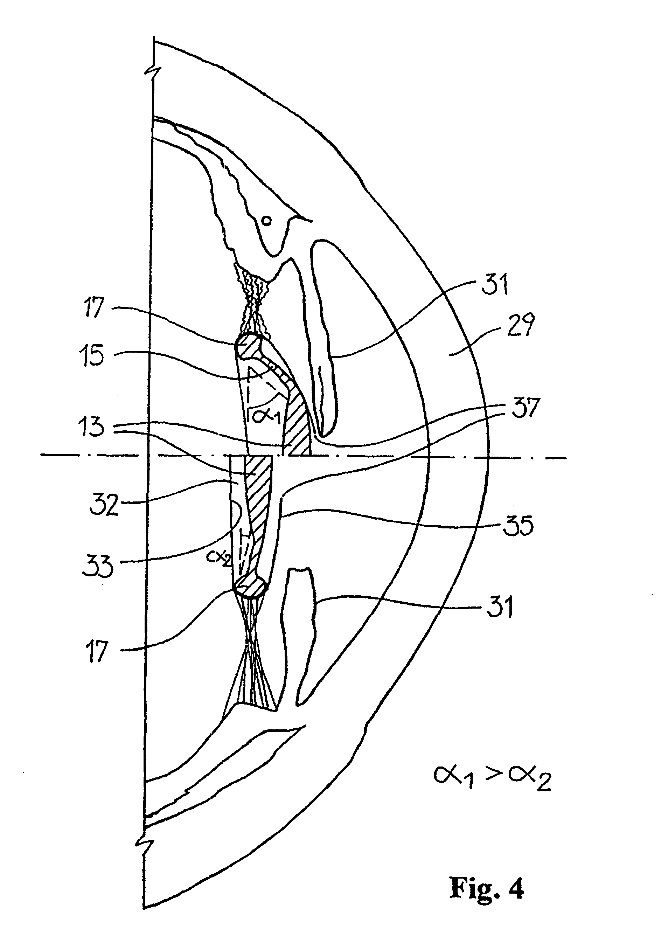

Fig. 4 shows only the portion of the human eye which adjoins the cornea 29. The iris 31 is behind the cornea and the lens capsule 33 is behind the iris. The lens 13 is inserted into the lens capsule 32 with the rear leaf 33 and the front leaf 35 and stretches it to some extent. In the state of near accommodation the lens 13 is pushed forward by the centripetal force of the elastic ciliary ring (Fig. 4, top half). The round window made in the front lens leaf 35 is marked 37.

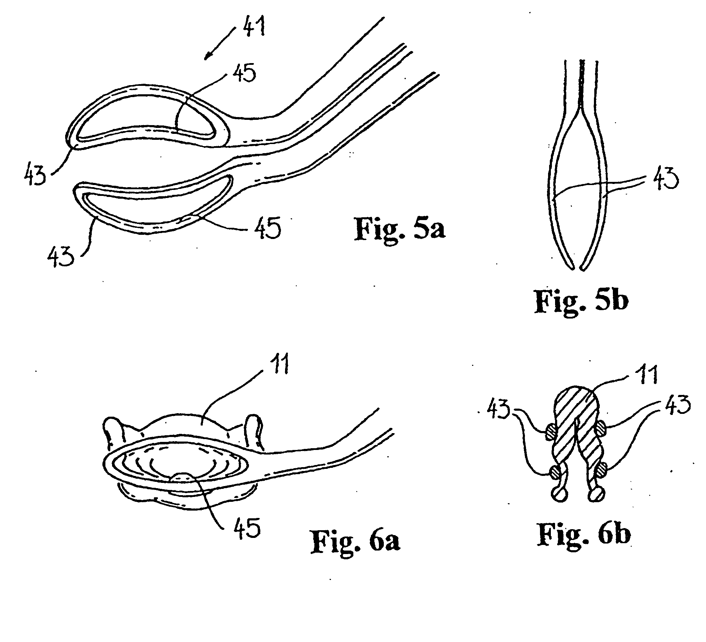

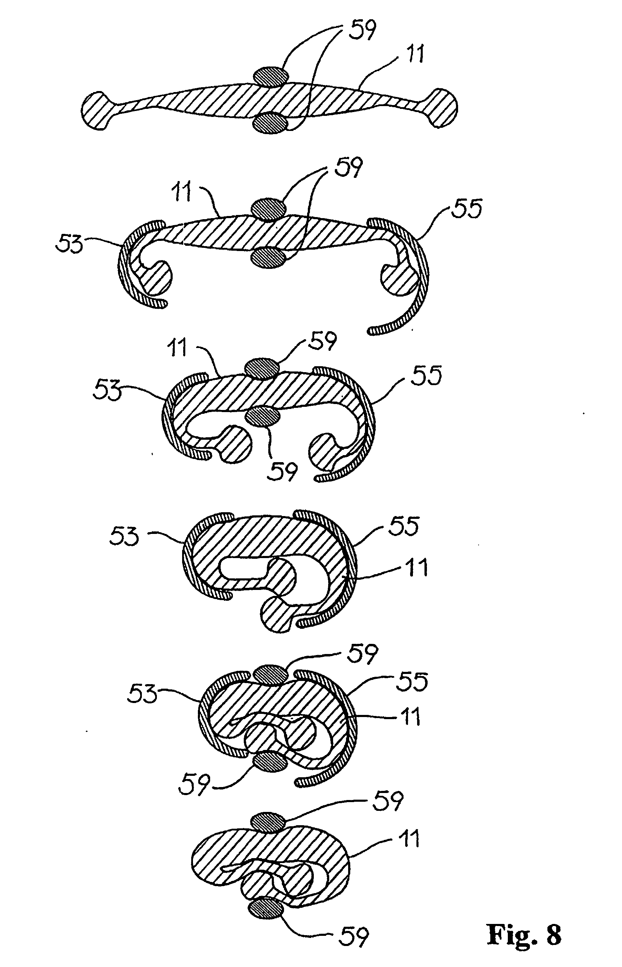

The folding forceps 41 has two gripping parts 43 movable in opposite directions, gripping in pairs, concave as seen from above, and touching at front and rear when in the closed state (Fig. 5b). The gripping parts are oval, each with an elongate recess 45 (Fig. 5a). When the elastic implant is compressed during the folding process, it swells into the recesses 45 and is thus held firmly during the implantation process (Fig. 6).

The embodiment of an implant 11 as shown in Fig. 1 has an outer diameter of 9.8 to 10 mm. The lens 13 has a diameter of 5.8 mm and the circular recesses 23 have a diameter of 1 mm. The intermediate region has a thickness of only about 0.2 mm. In the central region the lens, depending on the dioptric power, has a thickness of about 0.7 to about 1.35 mm (0.9 mm in the example shown). The peripheral width of the recesses is about 0.8 mm each.

The exemplified embodiment shown in Fig. 2 has a central lens 28 of increased refractive power, which improves near sight. Since the miotic convergence reaction of the pupils is retained during near accommodation of the two eyes and since older people frequently have a senile miosis, the eye acquires depth of focus like a pinhole camera, by screening out extra-axial rays (stenopaic principle).

The lens 13 is made of a bio-compatible material. Very good results have been obtained with a straight-chain high-molecular acrylic copolymer, e.g. of methyl acrylate and 2-hydroxyethyl methacrylate. Crosslinking agents, UV absorbers and starters are preferably fully incorporated in the polymer matrix, preventing any leaching. A particularly suitable material of the said kind is obtainable from Messrs Marcher GmbH, Stuttgart.

In the implantation process, a round window 37 is first traced in the front capsule wall 35, with a circular central capsulorhexis, and the lens capsule 32 is emptied by removing the cloudy lens masses. In a perfect operation, the lens capsule 32 and the zonule of Zinn are undamaged, thus avoiding injury to the corneal endothelium, which would have serious consequences. The implant is then folded, using the special folding forceps 41, so that the narrower edge bead portion 21' is at the rear in the direction of insertion, as shown by the broken line 27 in Fig. 2. The implant can then be partly inserted into the lens capsule 32, by stretching the round window, and can automatically slide after release, when it gently unfolds and is automatically centred.

The compression force for provoking an axial displacement of the lense have been determined experimentally according to ISO/DIS 11979-3 by using a miniature material tester (Vitrodyne V-200L) and a Laser scan micrometer from Keyence with a resolution of ±0.001 mm. The measurements were conducted in an aquous 1% NaCl-solution at 35 °C. The force necessary for provoking an axial displacement of the lense of 0.1 mm ranged between 0.85 mN and 0.65 mN per 0.1 mm displacement (see table).

| displacement in mm | force (in mN) |
|---|---|
| 0.1 - 0.5 | 0.85 |
| 0..5 - 1.5 | 0.65 |

The invention is not restricted to the embodiments shown. For example the zones of increased refractive power in Figs. 2a, 2b can be omitted or another kind of lens, such as a multi-focal lens, can be used. The important thing is that the haptic portion has at least one recess extending inwards from the periphery, the intermediate region being made elastically deformable so that when centripetal force is exerted on the edge bead there is practically no deformation of the lens, and instead the lens moves axially relative to the edge bead, i.e. forwards in the direction of the optical axis.

## Claims

1. An ocular implant for insertion into the lens capsule of an eye, comprising a lens (13) which is substantially non-deformable by the forces prevailing in the eye, and a haptic portion (15, 17) connected to the lens (13), the haptic portion having one or more recesses (19) extending inwards from the the periphery,
**characterised in that**
the ocular implant comprises a single lens, the haptic portion (15,17) thereof having an annular edge bead (17) and an annular intermediate region (15) being bent relatively to a plane given by the annular edge bead (17) and connecting the edge bead (17) to the lens (13), and **in that** the intermediate portion portion is constructed so as to be so elastically deformable that when centripetal pressure is exerted on the edge bead (17) of the implanted ocular implant there is substantially no deformation of the lens (13), but instead the lens (13) is moved axially forwardly relative to the edge bead (17).

2. An implant according to claim 1, **characterised in that** at least two approximately opposite recesses (19) are formed on the haptic portion.

3. An implant according to claim 1 or 2, **characterised in that** the haptic portion has three recesses (19).

4. An implant according to claim 3, **characterised in that** two recesses (19) are disposed with mirror symmetry at an angle of 120 to 150°, preferably 140°, to the recess at the front in the direction of insertion.

5. An implant according to claim 3 or 4, **characterised in that** undercuts (25, 25') are formed at the ends of the beads (17) at the front in the direction of insertion.

6. An implant according to any of claims 1 to 5, **characterised in that** the implant is foldable.

7. An implant according to any of claims 1 to 6, **characterised in that** the folding axis extends through at least one recess (19).

8. An implant according to any one of claims 1 to 7, **characterised in that** the intermediate region (15) has a number of spaced-apart recesses.

9. An implant according to any of claims 1 to 8, **characterised in that** the intermediate region (15), as a result of the recesses, is equivalent to a spoke structure.

10. An implant according to any of claims 1 to 9, **characterised in that** the haptic portion is bent forwards between about 5° and 15°, preferably about 10°, relative to a plane at right angles to the optical axis.

11. An implant according to any of claims 1 to 10, **characterised in that** the lens (13) has a central zone (28) of increased refractive power, i.e. is constructed as a bifocal lens (13) or as a multi-focal lens.

12. An implant according to claim 11, **characterised in that** the zone of increased refractive power is an approximately circular lens (28), e.g. about 0.8 to 2 mm in diameter, preferably about 1 mm.

13. An implant according to any of claims 1 to 13, **characterised in that** the lens body is biconvex and the haptic portion is substantially circular.

14. An implant according to any of claims 1 to 13, **characterised in that** the implant consists mainly of a methyl acrylate and 2-hydroxyethyl methacrylate copolymer.

## Patentansprüche

1. Okulares Implantat zum Einsetzen in den Kapselsack eines Auges, mit einer Linse (13), welche durch die im Auge vorherrschenden Kräften im Wesentlichen nicht deformierbar ist, und einer mit der Linse (13) verbundenen Haptik (15,17), welche Haptik eine oder mehrere von der Peripherie nach innen verlaufende Aussparungen (19) hat, **dadurch gekennzeichnet, dass** das okulare Implantat eine einzelne Linse aufweist, die damit verbundene Haptik (15,17) einen ringförmigen Randwulst (17) und einen ringförmigen Zwischenbereich aufweist, welcher bezüglich einer durch den ringförmigen Randwulst (17) gehenden Ebene gewölbt ist und den Randwulst (17) mit der Linse (13) verbindet, und dass der Zwischenbereich derart elastisch deformierbar ausgestaltet ist, dass bei zentripetalem Druck auf den Randwulst (17) keine Verformung der Linse (13) erfolgt, sondern die Linse (13) in bezug auf den Randwulst (17) axial nach vorne bewegt wird.

2. Implantat nach Anspruch 1, **dadurch gekennzeichnet, dass** an der Haptik zwei einander ungefähr gegenüberliegende Aussparungen (19) vorgesehen sind.

3. Implantat nach Anspruch 1 oder 2, **dadurch gekennzeichnet, dass** die Haptik drei Aussparungen (19) aufweist.

4. Implantat nach Anspruch 3, **dadurch gekennzeichnet, dass** zwei Aussparungen (19) zu der in Einführungsrichtung vorne liegenden Aussparung spiegelsymmetrisch in einem Winkel von 120 bis 150 Grad, vorzugsweise 140 Grad, angeordnet sind.

5. Implantat nach Anspruch 3 oder 4, **dadurch gekennzeichnet, dass** bei den Enden der bezüglich der Einführungsrichtung vorderen Randwulste (17) Hinterschneidungen (25,25') ausgebildet sind.

6. Implantat nach einem der Ansprüche 1 bis 5, **dadurch gekennzeichnet, dass** das Implantat faltbar ist.

7. Implantat nach einem der Ansprüche 1 bis 6, **dadurch gekennzeichnet, dass** die Faltachse durch wenigstens eine Aussparung (19) verläuft.

8. Implantat nach einem der Ansprüche 1 bis 7, **dadurch gekennzeichnet, dass** der Zwischenbereich (15) mehrere in Abstand voneinander angeordnete Ausnehmungen besitzt.

9. Implantat nach einem der Ansprüche 1 bis 8, **dadurch gekennzeichnet, dass** der Zwischenbereich (15) durch die Ausnehmungen einer Speichenstruktur gleichkommt.

10. Implantat nach einem der Ansprüche 1 bis 9, **dadurch gekennzeichnet, dass** die Haptik bezüglich einer zur optischen Achse senkrechten Ebene zwischen ungefähr 5° und 15°, vorzugsweise ungefähr 10°, nach vorne gewinkelt ist.

11. Implantat nach einem der Ansprüche 1 bis 10, **dadurch gekennzeichnet, dass** die Linse (13) im Zentrum eine Zone (28) erhöhter Brechkraft aufweist, d.h. als bifokale oder multifokale Linse (13) ausgebildet ist.

12. Implantat nach Anspruch 11, **dadurch gekennzeichnet, dass** die Zone erhöhter Brechkraft eine ungefähr kreisförmige Linse (28), beispielsweise von ungefähr 0.8 bis 2 mm , vorzugsweise von ungefähr 1 mm Durchmesser, ist.

13. Implantat nach einem der Ansprüche 1 bis 13, **dadurch gekennzeichnet, dass** der Linsenkörper bikonvex und die Haptik im Wesentlichen kreisrund ist.

14. Implantat nach einem der Ansprüche 1 bis 13, **dadurch gekennzeichnet, dass** das Implantat im Wesentlichen aus einem Copolymer aus Mehtylacrylat und 2-Hydroxyethylmethacrylat besteht.

## Revendications

1. Implant oculaire pour insertion dans la capsule de lentille d'un oeil comprenant une lentille (13) qui est essentiellement indéformable par les forces qui règnent dans l'oeil et une portion tactile (15, 17) reliée à la lentille (13), la portion tactile ayant un ou plusieurs évidements (19) qui s'étendent vers l'intérieur à partir de la périphérie,
**caractérisé en ce**
**que** l'implant oculaire comprend une seule lentille, la portion tactile (15, 17) de celle-ci ayant un bourrelet de bord annulaire (17) et une région intermédiaire annulaire (15) qui sont courbées par rapport à un plan donné par le bourrelet de bord annulaire (17) et reliant le bourrelet de bord (17) à la lentille (13) et en ce que la portion intermédiaire est construite de manière à être déformable élastiquement de telle manière que, lorsqu'une pression centripète est exercée sur le bourrelet de bord (17) de l'implant oculaire implanté, il n'y a substantiellement pas de déformation de la lentille (13) mais, à la place, la lentille (13) est déplacée axialement en avant par rapport au bourrelet de bord (17).

2. Implant selon la revendication 1, **caractérisé en ce qu'**au moins deux évidements approximativement opposés (19) sont formés sur la portion tactile.

3. Implant selon la revendication 1 ou 2, **caractérisé en ce que** la portion tactile a trois évidements (19).

4. Implant selon la revendication 3, **caractérisé en ce que** deux évidements (19) sont disposés en symétrie spéculaire à un angle de 120 à 150°, de préférence de 140°, par rapport à l'évidement sur le devant dans le sens de l'insertion.

5. Implant selon la revendication 3 ou 4, **caractérisé en ce que** des dépouilles (25, 25') sont formées aux extrémités des bourrelets (17) sur le devant dans le sens de l'insertion.

6. Implant selon l'une quelconque des revendications 1 à 5, **caractérisé en ce que** l'implant est pliable.

7. Implant selon l'une quelconque des revendications 1 à 6, **caractérisé en ce que** l'axe de pliage s'étend à travers au moins un évidement (19).

8. Implant selon l'une quelconque des revendications 1 à 7, **caractérisé en ce que** la région intermédiaire (15) a un certain nombre d'évidements espacés.

9. Implant selon l'une quelconque des revendications 1 à 8, **caractérisé en ce que** la région intermédiaire (15), en tant que résultat des évidements, est équivalent à une structure de rayons.

10. Implant selon l'une quelconque des revendications 1 à 9, **caractérisé en ce que** la portion tactile est courbée en avant entre environ 5° et 15°, de préférence d'environ 10°, par rapport à un plan à angles droits avec l'axe optique.

11. Implant selon l'une quelconque des revendications 1 à 10, **caractérisé en ce que** la lentille (13) a une zone centrale (28) de pouvoir réfractif accru, c'est-à-dire qu'elle est construite comme une lentille bifocale (13) ou une lentille à plusieurs foyers.

12. Implant selon la revendication 11, **caractérisé en ce que** la zone de pouvoir réfractif accru est une lentille approximativement circulaire (28), par exemple d'environ 0,8 à 2 mm de diamètre, de préférence d'environ 1 mm.

13. Implant selon l'une quelconque des revendications 1 à 13, **caractérisé en ce que** le corps de la lentille est biconvexe et la portion tactile est substantiellement circulaire.

14. Implant selon l'une quelconque des revendications 1 à 13, **caractérisé en ce que** l'implant consiste principalement en acrylate de méthyle et en copolymère de 2-hydroxyéthyl-méthacrylate.
